# EUROPÄISCHE PATENTSCHRIFT

(11) **EP 0 837 062 B1**
(45) Veröffentlichungstag und Bekanntmachung des Hinweises auf die Patenterteilung: **03.12.2003**
(21) Anmeldenummer: 97117642.5
(22) Anmeldetag: 11.10.1997
(51) Int. Cl.: C07D 317/36, C07D 317/72

(54) **Herstellung von Cyclocarbonaten**
Preparation of cyclocarbonates
Préparation de cyclocarbonates

(30) Priorität: 21.10.1996 EP 96116895
(43) Veröffentlichungstag der Anmeldung: 22.04.1998
(73) Patentinhaber: F. HOFFMANN-LA ROCHE AG, 4070 Basel (CH)
(72) Erfinder: Gründler, Hansjörg, 4310 Rheinfelden (CH); Hansen, Hans-Jürgen, 8006 Zürich (CH)
(74) Vertreter: Kellenberger, Marcus, Dr.

(56) Entgegenhaltungen:
- EP-A- 0 175 241
- DE-B- 1 098 953

## Beschreibung

### Herstellung von Cyclocarbonaten

Die vorliegende Erfindung betrifft ein verbessertes Verfahren zur Herstellung von cyclischen Carbonaten (Cyclocarbonaten) aus Prop-1-in-3-olen und Kohlendioxid in Gegenwart von Katalysatoren. Diese Cyclocarbonate, von denen das strukturell einfachste Mitglied das 4,4-Dimethyl-5-methylen-1,3-dioxolan-2-on (auch als "Cyclocarbonat" selber bekannt) ist, sind wertvolle Zwischenprodukte zur Herstellung von Polymerisaten und weiteren nützlichen Stoffen, z.B. Farbstoffen und Carotinoiden.

Aus der Fachliteratur sind verschiedene Verfahren zur Herstellung von Cyclocarbonaten, d.h. 4,4-disubstituierten 5-Methylen-1,3-dioxolan-2-onen der allgemeinen Formel 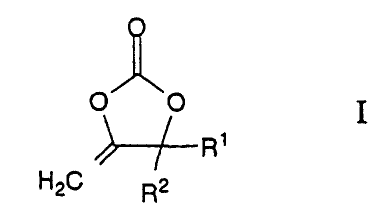 (später näher erläutert), bekannt geworden, die auf der katalysierten Reaktion des entsprechend 3,3-disubstituierten Prop-1-in-3-ols der allgemeinen Formel 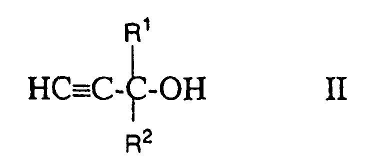 mit Kohlendioxid basieren. So werden als Katalysatoren unter anderen Kupfersalze, insbesondere Kupferhalogenide, z.B. Kupfer(II)chlorid oder Kupfer(I)jodid, oder Kupfer(II)acetat [siehe Deutsche Patentschrift 1.098.953, Synthesis 1981, 958-959 und Europäische Patentpublikation (EP) 175 241], Diacetonitrilopalladiumdichlorid (N. Yanagihara et al., 52^{nd} National Meeting of the Chemical Society of Japan, Kyoto April 1986, Abstr. No. 4W 15), Rutheniumtrichlorid-Trihydrat zusammen mit Triethylamin [Tetr. Lett. 27, Nr. 14, 1573-1574 (1986)], Dicyclopentadienylcobalt, ebenfalls zusammen mit Triethylamin [Bull. Chem. Soc. Japan 60, 1204-1206 (1987)] sowie Tributylphosphin [Tetr. Lett. 30, Nr. 30, 3981-3982 (1989)] verwendet. Es ist bis jetzt nicht bekannt, dass auch Silbersalze diese Reaktion katalysieren.

Es wurde nun gefunden, dass die obige Reaktion, welche zu den 4,4-disubstituierten 5-Methylen-1,3-dioxolan-2-onen der allgemeinen Formel I führt, auch durch Silbersalze katalysiert werden kann, wobei auch - wie in der EP 175 241 vorgesehen - ein quartäres Ammonium- oder Phosphoniumsalz als weiterer Katalysator verwendet wird. Im Gegensatz zur Lehre aus EP 175 241 wird allerdings eine tertiäre Base als noch weiterer Katalysator für dieses Verfahren nicht verwendet, oder deren Verwendung erübrigt sich. Hauptsächlich unterscheidet sich die vorliegende Erfindung von derjenigen der EP 175 241 jedoch dadurch, dass man als Katalysator anstelle eines Kupfersalzes ein Silbersalz verwendet.

In diesem Sinne handelt es sich bei der vorliegenden Erfindung um ein Verfahren zur Herstellung von 4,4-disubstituierten 5-Methylen-1,3-dioxolan-2-onen der allgemeinen Formel 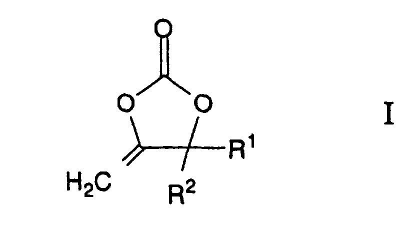 worin R¹ und R² unabhängig voneinander eine gesättigte oder olefinisch ungesättigte aliphatische Gruppe oder eine aromatische Gruppe bedeuten, oder R¹ und R² zusammen Tetra- oder Pentamethylen bilden,
durch Umsetzung eines entsprechend 3,3-disubstituierten Prop-1-in-3-ols der allgemeinen Formel 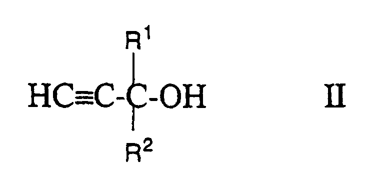 mit Kohlendioxid in Gegenwart eines quartären Ammonium- oder Phosphoniumsalzes als Katalysator, das dadurch gekennzeichnet ist, dass man als weiteren Katalysator ein Silbersalz verwendet.

In der obigen Definition des erfindungsgemässen Verfahrens sind unter dem Ausdruck "eine gesättigte aliphatische Gruppe" insbesondere Alkylgruppen mit 1 bis 16 Kohlenstoffatomen, die sowohl geradkettig als auch (ab C₃) verzweigt sein können, zu verstehen. Auch die "olefinisch ungesättigte aliphatische Gruppe" (eine Alkenylgruppe) enthält insbesondere bis zu 16 Kohlenstoffatome und kann geradkettig oder verzweigt sein. Diese Gruppe kann zudem mono- oder mehrfach ungesättigt sein, wobei mehrere Doppelbindungen konjugiert oder nicht-konjugiert sein können. Beispiele dieser Alkyl- und Alkenylgruppen sind Methyl, Ethyl, Isopropyl, Isobutyl, tert.Butyl und 4,8-Dimethyl-nonyl bzw. Vinyl, 1-Propenyl, Allyl, 4-Methyl-3-pentenyl, 4,8-Dimethyl-1,7-nonadienyl und 4,8-Dimethyl-1,3,7-nonatrienyl. Als "aromatische Gruppe" kommt insbesondere Phenyl oder Naphthyl in Frage, welches jeweils gegebenenfalls substituiert ist, und zwar beispielsweise mit einer(m) oder mehreren (gleichen oder verschiedenen) C₁₋₄-Alkyl- und C₂₋₄-Alkenylgruppen und Halogenatomen. Beispiele der aromatischen Gruppe sind Phenyl, p-Toluyl und Naphthyl.

Bedeuten R¹ und R² zusammen Tetra- oder Pentamethylen, so liegt zusammen mit dem Kohlenstoffatom, an das R¹ und R² gebunden sind, ein Cyclopentan- bzw. Cyclohexanring vor.

Als erfindungsgemäss verwendete Silbersalze eignen sich im Prinzip alle, wie beispielsweise Silberacetat, Silbersulfat, Silberorthophosphat, Silberoxid und Silbercarbonat. Vorzugsweise verwendet man als Silbersalz-Katalysator Silberacetat oder Silbersulfat.

Ebenfalls als Katalysator im erfindungsgemässen Verfahren wird ein quartäres Ammonium- oder Phosphoniumsalz verwendet. Dieses Salz weist zweckmässigerweise die allgemeine Formel

(R³)₄N⁺ Hal⁻ IIIa

bzw.

(R³)₄P⁺ Hal⁻ IIIb

worin jedes R³ unabhängig C₁₋₆-Alkyl, C₅₋₇-Cycloalkyl, Aryl-C₁₋₄alkyl oder Aryl und Hal Halogen bedeuten,
auf. In dieser Definition kann jede Alkylgruppe bzw. jeder Alkylenteil der Aryl-C₁₋₄-alkyl-Gruppe geradkettig oder verzweigt sein. Unter dem Ausdruck "Aryl" (als solches oder als Teil der Aryl-C₁₋₄-alkyl-Gruppe) sind insbesondere Phenyl und Naphthyl zu verstehen, wobei eine solche Gruppe unsubstituiert oder substituiert sein kann, und zwar zweckmässigerweise mit einer(m) oder mehreren (gleichen oder verschiedenen) C₁₋₄-Alkyl- und C₂₋₄-Alkenylgruppen und Halogenatomen. Vorzugsweise ist jedes C₁₋₆-Alkyl Methyl, Ethyl, Isopropyl, n-Butyl oder Hexyl, jedes C₅₋₇-Cycloalkyl Cyclopentyl oder Cyclohexyl, jedes Aryl-C₁₋₄-alkyl Benzyl bzw. jedes Aryl unsubstituiertes Phenyl oder Naphthyl. Hal ist vorzugsweise Chlor oder Brom. Besonders bevorzugte Beispiele des quartären Ammoniumsalzes der Formel IIIa sind Tetramethylammoniumchlorid, Tetraethylammoniumchlorid, Tetraethylammoniumbromid und Benzyltrimethylammoniumchlorid, bzw. des quartären Phosphoniumsalzes der Formel IIIb sind Tetrabutylphosphoniumbromid, Tetraphenylphosphoniumchlorid und Tetraphenylphosphoniumbromid.

Als Kohlendioxid verwendet man im erfindungsgemässen Verfahren gasförmiges Kohlendioxid, welches zweckmässigerweise in das bereits die restlichen Reaktionsteilnehmer enthaltende Reaktionsgefäss, geeigneterweise einen Druckautoklaven, eingeleitet wird. Eine Erhöhung des Kohlendioxid-Drucks über Normaldruck erhöht die Reaktionsgeschwindigkeit. Daher wird das Verfahren zweckmässigerweise unter einem Kohlendioxid-Druck durchgeführt, der oberhalb von 1 bar (100 kPa), vorzugsweise im Bereich von etwa 1 bar bis etwa 30 bar (etwa 100 kPa bis etwa 3000 kPa), liegt. Bei Verwendung von Silberacetat als Silbersalz-Katalysator liegt der bevorzugte Druck im Bereich von etwa 15 bar bis etwa 25 bar (etwa 1500 kPa bis etwa 2500 kPa).

Des weiteren kann das erfindungsgemässe Verfahren gewünschtenfalls in einem inerten Lösungsmittel erfolgen. Dazu eignet sich im allgemeinen ein polares protisches oder aprotisches Lösungsmittel, insbesondere ein aliphatischer Kohlenwasserstoff, z.B. n-Hexan; ein aliphatischer Ether, z.B. tert.Butylmethylether; ein aliphatisches Keton, z.B. 2-Methylbutan-3-on; ein aliphatisches Amid, z.B. Dimethylformamid; oder ein aromatischer Kohlenwasserstoff, z.B. Toluol. Bevorzugte Lösungsmittel sind die aliphatischen und aromatischen Kohlenwasserstoffe sowie die aliphatischen Ether. Im allgemeinen ist die Verwendung eines Lösungsmittels angezeigt, wenn das Edukt und/oder das Produkt unter den Reaktionsbedingungen fest sind (da dessen bzw. deren Schmelzpunkt(e) zu hoch ist bzw. sind). Falls nicht wegen ungenügender Löslichkeit des Edukts und/oder des Produktes notwendig, wird das erfindungsgemässe Verfahren vorzugsweise ohne Einsatz eines Lösungsmittels durchgeführt.

Was die Mengenverhältnisse betrifft, verwendet man pro Mol Edukt der Formel II zweckmässigerweise etwa 0,05 bis etwa 0,3 Mol-% Silbersalz-Katalysator und etwa 0,05 bis etwa 0,3 Mol-% Oniumsalz-Katalysator (quartäres Ammoniumsalz der Formel IIIa und/oder quartäres Phosphoniumsalz der Formel IIIb).

Das erfindungsgemässe Verfahren erfolgt zweckmässigerweise im Temperaturbereich von etwa 25°C (also bei Raumtemperatur) bis etwa 100°C.

Die katalytische Leistung der verschiedenen Silbersalze ist unterschiedlich. Es wurde im Zusammenhang mit der vorliegenden Erfindung ferner gefunden, dass die Leistung der eher weniger katalytisch aktiven Silbersalze, wie beispielsweise Silberorthophosphat, Silberoxid und Silbercarbonat, durch Zusatz eines Alkalimetall- oder quartären Ammoniumoder Phosphoniumsalzes einer Carbonsäure gesteigert werden kann, wobei das diesbezügliche quartäre Ammonium- oder Phosphoniumion insbesondere das obenerwähnte und definierte Ion (R³)₄N⁺ bzw. (R³)₄P⁺ ist. Zu diesem Zweck gibt man geeigneterweise eine etwa äquimolare Menge des Carbonsäuresalzes bezüglich der Menge Silbersalz zu. Beispiele der in Frage kommenden Carbonsäuresalze sind Natriumacetat, Malonsäuremonoethylester-Kaliumsalz, Maleinsäure-Dinatriumsalz sowie Tetraethylammoniumacetat. Die Alkalimetall- und quartären Ammonium- und Phosphoniumacetate sind bevorzugt.

Ein weiteres Merkmal der vorliegenden Erfindung besteht darin, das Verfahren auch in Gegenwart von Triphenylphosphin durchzuführen. Der Zusatz von Triphenylphosphin dient dazu, die Reaktion auch etwas zu beschleunigen, und ist deshalb vorteilhaft. Man verwendet pro Mol Edukt der Formel II zweckmässigerweise bis zu etwa 0,2 Mol-% Triphenylphosphin.

Im allgemeinen kann man das erfindungsgemässe Verfahren auf eine kontinuierliche Arbeitsweise oder als Eintopfverfahren (diskontinuierlich) vornehmen.

Die Aufarbeitung des nach Beendigung der Reaktion erhaltenen Gemisches zwecks Isolierung des erwünschten 4,4-disubstituierten 5-Methylen-1,3-dioxolan-2-ons der Formel I kann in an sich bekannter Weise erfolgen. Es hat sich als besonders praktisch erwiesen, das Cyclocarbonat aus dem Gemisch durch fraktionierte Destillation zu gewinnen. In den meisten Fällen können die katalysatorhaltigen Rückstände für weitere Reaktionsansätze verwendet werden bzw. in die Bestandteile aufgeteilt und diese dann weiterverwendet werden.

Bei thermisch unstabilen Cyclocarbonaten kann die Reinigung durch Chromatographie erfolgen.

Der Vorteil des erfindungsgemässen Verfahrens gegenüber den bisher bekannten Verfahren zur Herstellung von Cyclocarbonaten besteht vor allem darin, dass die Silbersalze die diesbezügliche Reaktion deutlich besser katalysieren als beispielsweise die Kupfersalze. Dies äussert sich dadurch, dass die Reaktion durch viel kleinere Mengen Katalysator katalysiert wird, die Reaktionszeit deutlich kürzer ist, und weniger drastische Reaktionsbedingungen für die gute Reaktionszeit nötig sind.

Die Erfindung wird anhand der nachfolgenden Beispiele veranschaulicht:

### Beispiel 1

### Herstellung von 4,4-Dimethyl-5-methylen-1,3-dioxolan-2-on

In einem mit Manometer, Thermometer, Sicherheitsventil und Anschlüssen für Kohlendioxid-Begasung ausgestatteten Stahlautoklaven werden 54,68 g (0,65 Mol) 2-Methyl-3-butin-2-ol vorgelegt. Dazu werden 107 mg (0,41 mmol, 0,063 Mol-%) Triphenylphosphin, 68 mg (0,41 mmol, 0,063 Mol-%) Silberacetat sowie 137 mg (0,81 mmol, 0,125 Mol-%) Tetraethylammoniumchlorid gegeben. Dann wird der Autoklav verschlossen und mit Kohlendioxid gespült, und zwar dadurch, dass bei ausgeschaltetem Rührer Kohlendioxid bis zu einem Druck von etwa 20 bar (2000 kPa) aufgedrückt und anschliessend wieder entspannt wird; der Zyklus Aufdrücken und Entspannung wird dreimal durchgeführt.

Anschliessend werden 15 bar (1500 kPa) Kohlendioxid aufgedrückt, und das Reaktionsgemisch wird unter Rühren innert etwa 20 Minuten auf 80°C erwärmt. Ist die Innentemperatur von 80°C erreicht, wird der Innendruck bis auf 20 bar (2000 kPa) erhöht. Das Gemisch wird bei 20 bar (2000 kPa) und 80°C weitergerührt. Nach 3 Stunden wird der Druck auf 5 bar (500 kPa) entspannt und die Temperatur auf 40°C gesenkt. Ist eine Innentemperatur von 40°C erreicht, wird vorsichtig auf Normaldurck entspannt. (Unter Druck löst sich sehr viel Kohlendioxid im Produkt, so dass der Druck deshalb sehr langsam reduziert werden muss, um ein Aufschäumen des Reaktionsgemisches zu verhindern.) Da der Schmelzpunkt des so produzierten Cyclocarbonats über 30°C liegt, darf nicht bis auf Raumtemperatur abgekühlt werden.

Man überträgt dann das Gemisch in eine Destillationsapparatur, wo es mittels Vakuumdestillation fraktioniert wird. Die Innentemperatur wird zunächst auf 40°C erhöht und dabei die Apparatur vorsichtig evakuiert. Das noch teilweise gelöste Kohlendioxid perlt dabei aus. Nachdem die Apparatur bis auf etwa 10 mbar (1 kPa) evakuiert worden ist, wird die Temperatur des Oelbades langsam auf 100°C erhöht. Dabei beginnt das Produkt zu destillieren. Kopftemperatur 67-69°C, Druck 12 mbar (1,2 kPa).

Nach beendeter Destillation bleibt ein schwarzer Rückstand zurück, den man in Methylenchlorid auflöst und entsorgt.

Auf diese Weise erhält man 78,59 g (98,66%iger Gehalt) 4,4-Dimethyl-5-methylen-1,3-dioxolan-2-on; die Ausbeute beträgt 93,1% bezogen auf eingesetztes Methylbutinol.

### Beispiel 2

### Herstellung von 4-Ethyl-4-methyl-5-methylen-1,3-dioxolan-2-on

Ausgehend von 63,79 g (0,65 Mol) 3-Methyl-1-pentin-3-ol werden nach der in Beispiel 1 beschriebenen Methodik 74,54 g (99,99%iger Gehalt) 4-Ethyl-4-methyl-5-methylen-1,3-dioxolan-2-on erhalten; die Ausbeute beträgt 80,67% bezogen auf eingesetztes Methylpentinol.

### Beispiel 3

### Herstellung von 4-Methyl-4-(4-methyl-3-pentenyl)-5-methylen-1,3-dioxolan-2-on

Ausgehend von 68,51 g (0,45 Mol) 3,7-Dimethyl-oct-1-in-6-en-3-ol (Dehydrolinalool) werden nach der in Beispiel 1 beschriebenen Methodik - allerdings mit einer 8-stündigen Erhitzenszeit bei 20 bar (2000 kPa) und 80°C - 69,41 g (97,79%iger Gehalt) 4-Methyl-4-(4-methyl-3-pentenyl)-5-methylen-1,3-dioxolan-2-on erhalten; die Ausbeute beträgt 76,9% bezogen auf eingesetztes Dehydrolinalool.

### Beispiel 4

### Herstellung von 4-Methylen-1,3-dioxa-spiro-[4,5]-decan-2-on

Ausgehend von 80,82 g (0,65 Mol) 1-Ethinyl-1-cyclohexanol werden nach der in Beispiel 1 beschriebenen Methodik - allerdings mit einer 5-stündigen Erhitzenszeit bei 20 bar (2000 kPa) und 80°C - 78,59 g (99,99%iger Gehalt) 4-Methylen-1,3-dioxa-spiro-[4.5]-decan-2-on erhalten; die Ausbeute beträgt 71,2% bezogen auf eingesetztes Ethinylcyclohexanol.

### Beispiel 5

### Herstellung von 4-Methyl-4-(2-methyl-propyl)-5-methylen-1,3-dioxolan-2-on

Ausgehend von 70,0 g (0,549 Mol) 3,5-Dimethyl-hex-1-in-3-ol werden nach der in Beispiel 1 beschriebenen Methodik - allerdings mit einer 8-stündigen Erhitzenszeit bei 20 bar (2000 kPa) und 80°C - 60,15 g (99,7%iger Gehalt) 4-Methyl-4-(2-methyl-propyl)-5-methylen-1,3-dioxolan-2-on erhalten; die Ausbeute beträgt 62,17% bezogen auf eingesetztes 3,5-Dimethyl-hex-1-in-3-ol.

### Beispiel 6

### Herstellung von 4-Methyl-5-methylen-4-vinyl-1,3-dioxolan-2-on

Ausgehend von 85,45 g (0,800 Mol) 3-Methyl-pent-1-en-4-in-3-ol werden nach der in Beispiel 1 beschriebenen Methodik - allerdings mit einer 5-stündigen Erhitzenszeit bei 20 bar (2000 kPa) und 80°C - 86,23 g (98,4 %iger Gehalt) 4-Methyl-5-methylen-4-vinyl-1,3-dioxolan-2-on erhalten; die Ausbeute beträgt 75,7% bezogen auf eingesetztes 3-Methyl-pent-1-en-4-in-3-ol.

### Beispiel 7

### Herstellung von 4,4-Diethyl-5-methylen-1,3-dioxolan-2-on

Ausgehend von 70,81 g (0,625 Mol) 3-Ethyl-1-pentin-3-ol werden nach der in Beispiel 1 beschriebenen Methodik - allerdings mit 0,25 Mol-% Triphenylphosphin, 0,25 Mol-% Silberacetat und 0,5 Mol-% Tetraethylammoniumchlorid und nach 21-stündigem Erhitzen bei 20 bar (2000 kPa) und 80°C - 78,13 g (96,5 %iger Gehalt) 4,4-Diethyl-5-methylen-1,3-dioxolan-2-on erhalten; die Ausbeute beträgt 77,2% bezogen auf eingesetztes 3-Ethyl-1-pentin-3-ol.

### Beispiel 8

### Herstellung von 4-Methyl-4-isopropyl-5-methylen-1,3-dioxolan-2-on

Ausgehend von 65,0 g (0,572 Mol) 2,3-Dimethyl-pent-4-in-3-ol werden nach der in Beispiel 1 beschriebenen Methodik - allerdings mit 0,25 Mol-% Triphenylphosphin, 0,25 Mol-% Silberacetat und 0,5 Mol-% Tetraethylammoniumchlorid und nach 4-stündigem Erhitzen bei 20 bar (2000 kPa) und 80°C - 73,8 g (98,7 %iger Gehalt) 4-Methyl-4-isopropyl-5-methylen-1,3-dioxolan-2-on erhalten; die Ausbeute beträgt 81,5% bezogen auf eingesetztes 2,3-Dimethyl-pent-4-in-3-ol.

### Beispiel 9

### Herstellung von 4-(4,8-Dimethyl-nonyl)-4-methyl-5-methylen-1,3-dioxolan-2-on

Ausgehend von 50,24 g (0,217 Mol) 3,7,11-Trimethyl-dodec-1-in-3-ol werden nach der in Beispiel 1 beschriebenen Methodik - allerdings mit 0,25 Mol-% Triphenylphosphin, 0,25 Mol-% Silberacetat und 0,5 Mol-% Tetraethylammoniumchlorid und nach 6-stündigem Erhitzen bei 20 bar (2000 kPa) und 80°C - 44,36 g (99 %iger Gehalt) 4-(4,8-Dimethyl-nonyl)-4-methyl-5-methylen-1,3-dioxolan-2-on erhalten; die Ausbeute beträgt 75,4% bezogen auf eingesetztes 3,7,11-Trimethyl-dodec-1-in-3-ol.

### Beispiel 10

### Herstellung von 4-(4,8-Dimethyl-nona-1,3,7-trienyl)-4-methyl-5-methylen-1,3-dioxolan-2-on

Ausgehend von 77,98 g (0,250 Mol) 3,7,11-Trimethyl-dodeca-4,6,10-trien-1-in-3-ol werden nach der in Beispiel 1 beschriebenen Methodik - allerdings mit 0,25 Mol-% Triphenylphosphin, 0,25 Mol-% Silberacetat und 0,5 Mol-% Tetraethylammoniumchlorid und nach 22-stündigem Erhitzen bei 25 bar (2500 kPa) und 60°C, Reinigung nicht durch Destillation sondern durch Säulenchromatographie an Kieselgel mit Hexan/Diisopropylether (17:1) als Laufmittel - 41,1 g (93 %iger Gehalt) 4-(4,8-Dimethyl-nona-1,3,7-trienyl)-4-methyl-5-methylen-1,3-dioxolan-2-on erhalten; die Ausbeute beträgt 58,3% bezogen auf eingesetztes 3,7,11-Trimethyl-dodeca-4,6,10-trien-1-in-3-ol.

### Beispiel 11

### Herstellung verschiedener 4,4-disubstituierter 5-Methylen-1,3-dioxolan-2-one unter Verwendung von Silber- oder Kupferacetat als Silber-/Kupfersalz-Katalysator: ein Vergleich

Die "Ethinyl-alkohole" 2-Methyl-3-butin-2-ol, 3-Methyl-1-pentin-3-ol, 2-Phenyl-3-butin-2-ol und 1-Ethinyl-1-cyclohexanol werden der Reihe nach mit Kohlendioxid unter Verwendung von Silber- oder Kupferacetat als Katalysator umgesetzt, und zwar nach folgender allgemeiner Arbeitsvorschrift:

In einem mit Manometer, Thermometer, Sicherheitsventil und Anschlüssen für Kohlendioxid-Begasung ausgestatteten Stahlautoklaven werden 650 mmol Ethinyl-alkohol vorgelegt. Dazu werden 0,41 mmol (0,063 Mol-%) Triphenylphosphin, 0,41 mmol (0,063 Mol-%) Silber- oder Kupferacetat und 0,82 mmol (0,125 Mol-%) Tetraethylammoniumchlorid gegeben. Der Autoklav wird verschlossen und mit Kohlendioxid gespült. Anschliessend werden 15 bar (1500 kPa) Kohlendioxid aufgedrückt, und das Reaktionsgemisch wird unter Rühren innert etwa 20 Minuten auf 80°C erwärmt. Ist die Innentemperatur von 80°C erreicht, wird der Innendruck bis auf 20 bar (2000 kPa) erhöht. Das Gemisch wird bei 20 bar (2000 kPa) und 80°C weitergerührt und jede Stunde eine Probe entnommen und mittels Gaschromatographie analysiert. Wenn die Reaktion beendet ist, wird der Druck auf 5 bar (500 kPa) entspannt und die Temperatur auf 40°C gesenkt. Ist eine Innnentemperatur von 40°C erreicht, wird vorsichtig auf Normaldruck entspannt.

Man überträgt das Reaktionsgemisch in eine Destillationsapparatur, wo es mittels Vakuumdestillation fraktioniert wird. Die Innentemperatur wird zunächst auf 40°C erhöht und dabei die Apparatur vorsichtig evakuiert. Das noch teilweise gelöste Kohlendioxid perlt dabei aus. Nachdem die Apparatur bis auf etwa 10 mbar (1 kPa) evakuiert worden ist, wird die Temperatur des Oelbades langsam erhöht, bis das Produkt zu destillieren beginnt.

Nach beendeter Destillation bleibt ein schwarzer Rückstand zurück, den man in Methylenchlorid auflöst und entsorgt.

Die Ergebnisse der Analysen sind in der nachfolgenden Tabelle 1 zusammengefasst:

**Tabelle 1**

| Carboxylierung von Ethinylalkoholen unter Katalyse durch Silber- oder Kupferacetat [AgOCOCH₃ bzw. Cu(OCOCH₃)₂] | | | | | | | |
|---|---|---|---|---|---|---|---|
| Edukt (Ethinyl-alkohol) | Katalysator | Prozentualer Gehalt (nach Gaschromatographie, Flächenprozent) an Cyclocarbonat im Reaktionsgemisch nach Stunden: | | | | | |
| | | 1 | 2 | 3 | 4 | 5 | 6 |
| 2-Methyl-3-butin-2-ol | AgOCOCH₃ | 92,6 | 98,3 | 99,5 | | | |
| | Cu(OCOCH₃)₂ | 36,8 | 66,9 | 83,0 | 89,4 | 93,1 | 94,7 |
| 3-Methyl-1-pentin-3-ol | AgOCOCH₃ | 74,9 | 95,1 | 100 | | | |
| | Cu(OCOCH₃)₂ | 17,0 | 35,4 | 51,2 | 65,8 | | |
| 2-Phenyl-3-butin-2-ol | AgOCOCH₃ | 46,4 | 60,6 | 64,0 | 66,6 | 67,7 | 67,7 |
| | Cu(OCOCH₃)₂ | 0,8 | 1,8 | 3,3 | 4,6 | 7,1 | 8,8 |
| 1-Ethinyl-1-cyclohexanol | AgOCOCH₃ | 54,2 | 77,4 | 92,0 | 98,4 | 99,6 | |
| | Cu(OCOCH₃)₂ | 5,0 | 8,3 | 15,6 | 31,9 | 32,8 | 41,5 |

### Beispiel 12

### Herstellung von 4,4-Dimethyl-5-methylen-1.3-dioxolan-2-on unter verschiedenen Reaktionsbedingungen

Nach der Arbeitsvorschrift von Beispiel 11 wurde jeweils 2-Methyl-3-bütin-2-ol mit Kohlendioxid (CO₂) unter Katalyse durch Silberacetat oder Kupferacetat umgesetzt. Dabei wurde das Verfahren einmal unter Standardbedingungen (bei 80°C und 20 bar (2000 kPa) CO₂-Druck), einmal unter Normaldruck (bei 80°C und 1 bar (100 kPa) CO₂-Druck) und einmal bei Raumtemperatur (bei 25°C und 20 bar (2000 kPa) CO₂-Druck) durchgeführt. Die Ergebnisse sind in der nachfolgenden Tabelle 2 zusammengefasst:

**Tabelle 2**

| Carboxylierung bei unterschiedlichen Reaktionstemperaturen und -drucken | | | | | | | |
|---|---|---|---|---|---|---|---|
| Bedingungen (Temperatur/Druck) | Katalysator | Prozentualer Gehalt (nach Gaschromatographie, Flächenprozent) an Cyclocarbonat im Reaktionsgemisch nach Stunden: | | | | | |
| | | 1 | 2 | 3 | 4 | 5 | 6 |
| 80°C/20 bar (2000 kPa) | AgOCOCH₃ | 92,6 | 98,3 | 99,5 | | | |
| | Cu(OCOCH₃)₂ | 36,8 | 66,9 | 83,0 | 89,4 | 93,1 | 94,7 |
| 80°C/1 bar (100 kPa)* | AgOCOCH₃ | 6,9 | 13,0 | 23,3 | 33,9 | 44,2 | 56,7 |
| | Cu(OCOCH₃)₂ | 3,3 | 8,3 | 11,6 | 19,7 | 25,0 | 33,4 |
| 25°C/20 bar (2000 kPa)* | AgOCOCH₃ | 7,7 | 11,8 | 15,6 | 19,6 | 23,0 | 26,7 |
| | Cu(OCOCH₃)₂ | 0,8 | 2,4 | 4,0 | 6,1 | 8,8 | 10,0 |

| | | | | | | | |
|---|---|---|---|---|---|---|---|
| * Um bei diesen Reaktionsbedingungen eine genügende Reaktionsgeschwindigkeit zu erhalten, wurde die Konzentration des Katalysatorgemisches erhöht, und zwar auf die achtfache Menge des Standardansatzes. | | | | | | | |

### Beispiel 13

### Herstellung von 4,4-Dimethyl-5-methylen-1,3-dioxolan-2-on unter Katalyse durch verschiedene Silbersalz-Katalysatoren

Nach der Arbeitsvorschrift von Beispiel 11 wurde jeweils 2-Methyl-3-butin-2-ol mit Kohlendioxid unter Katalyse durch verschiedene Silbersalz-Katalysatoren umgesetzt. Die Ergebnisse sind in der nachfolgenden Tabelle 3 zusammengefasst:

**Tabelle 3**

| Carboxylierung unter Katalyse durch verschiedene Silbersalz-Katalysatoren | | | |
|---|---|---|---|
| Katalysator | Prozentualer Gehalt (nach Gaschromatographie, Flächenprozent) an Cyclocarbonat im Reaktionsgemisch nach Stunden: | | |
| | 1 | 2 | 3 |
| Silbersulfat | 89,2 | 98,3 | 99,7 |
| Silberacetat | 87,7 | 98,7 | 100 |
| Silberorthophosphat | 37,4 | 46,3 | 51,3 |
| Silberoxid | 23,8 | 36,2 | 47,4 |
| Silbercarbonat | 18,0 | 24,8 | 35,9 |

### Beispiel 14

### Herstellung von 4,4-Dimethyl-5-methylen-1,3-dioxolan-2-on unter Katalyse durch Silbernitrat oder -trifluoracetat mit zugesetztem Carbonsäurealkalimetallsalz

Nach der Arbeitsvorschrift von Beispiel 11 wurde jeweils 2-Methyl-3-butin-2-ol mit Kohlendioxid unter Katalyse durch Silbernitrat oder - trifluoracetat und zugesetztes Carbonsäurealkalimetallsalz umgesetzt. Das Carbonsäurealkalimetallsalz wurde dabei jeweils in einer äquimolaren Menge bezogen auf die Menge Silbersalz eingesetzt, und die restlichen Katalysator-Komponenten in doppelter Menge. Die Ergebnisse sind in der nachfolgenden Tabelle 4 zusammengefasst:

**Tabelle 4**

| Carboxylierung unter Katalyse durch Silbernitrat oder -trifluoracetat und verschiedene Carbonsäurealkalimetallsalze | | | | |
|---|---|---|---|---|
| Zugesetztes Carbonsäure-alkalimetallsalz/Silbersalz | Prozentualer Gehalt (nach Gaschromatographie, Flächenprozent) an Cyclocarbonat im Reaktionsgemisch nach Stunden: | | | |
| | 1 | 2 | 3 | 4 |
| Natriumacetat/Silbernitrat | 84,6 | 98,8 | 99,4 | |
| Malonsäuremonoethylester-Kaliumsalz/Silbertrifluoracetat | 72,4 | 89,0 | 93,8 | 95,6 |
| Maleinsäure-Dinatriumsalz/ Silbertrifluoracetat | 15,1 | 27,0 | 44,0 | 54,1 |

### Beispiel 15

### Herstellung von 4.4-Dimethyl-5-methylen-1,3-dioxolan-2-on unter Verwendung von verschiedenen Lösungsmitteln

Nach der Arbeitsvorschrift von Beispiel 11 wurde jeweils 2-Methyl-3-butin-2-ol mit Kohlendioxid umgesetzt, allerdings unter zusätzlicher Verwendung eines Lösungsmittels. Pro Gramm Methylbutinol wurde 1,5 ml des Lösungsmittels zugesetzt. Die Ergebnisse sind in der nachfolgenden Tabelle 5 zusammengefasst:

**Tabelle 5**

| Carboxylierung unter Verwendung von verschiedenen Lösungsmitteln | | | | | | |
|---|---|---|---|---|---|---|
| Lösungsmittel | Prozentualer Gehalt (nach Gaschromatographie, Flächenprozent) an Cyclocarbonat im Reaktionsgemisch nach Stunden: | | | | | |
| | 1 | 2 | 3 | 4 | 6 | 8 |
| n-Hexan | 22,2 | 69,3 | 74,1 | 81,1 | 88,8 | 93,7 |
| tert.Butylmethylether | 27,4 | 56,2 | 76,2 | 81,4 | 89,5 | 91,9 |
| Toluol | 53,5 | 87,8 | 94,9 | 98,7 | 99,4 | 100 |

## Patentansprüche

1. Verfahren zur Herstellung von 4,4-disubstituierten 5-Methylen-1,3-dioxolan-2-onen der allgemeinen Formel 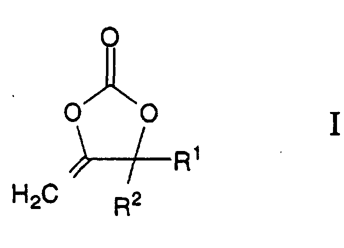 worin R¹ und R² unabhängig voneinander eine gesättigte oder olefinisch ungesättigte aliphatische Gruppe oder eine gegebenenfalls substituierte aromatische Gruppe bedeuten, oder R¹ und R² zusammen Tetra- oder Pentamethylen bilden,
durch Umsetzung eines entsprechend 3,3-disubstituierten Prop-1-in-3-ols der allgemeinen Formel 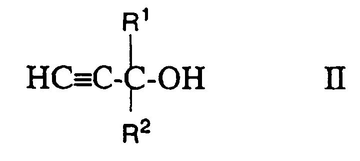 mit Kohlendioxid in Gegenwart eines quartären Ammonium- oder Phosphoniumsalzes als Katalysator, **dadurch gekennzeichnet ist, dass** man als weiteren Katalysator ein Silbersalz verwendet.

2. Verfahren nach Anspruch 1, **dadurch gekennzeichnet, dass** als Silbersalz-Katalysator Silberacetat, Silbersulfat, Silberorthophosphat, Silberoxid oder Silbercarbonat, vorzugsweise Silberacetat oder Silbersulfat, verwendet wird.

3. Verfahren nach Anspruch 1 oder 2, **dadurch gekennzeichnet, dass** als quartäres Ammoniumsalz Tetramethylammoniumchlorid, Tetraethylammoniumchlorid, Tetraethylammoniumbromid oder Benzyltrimethylammoniumchlorid, bzw. als quartäres Phosphoniumsalz Tetrabutylphosphoniumbromid, Tetraphenylphosphoniumchlorid oder Tetraphenylphosphoniumbromid verwendet wird.

4. Verfahren nach einem der Ansprüche 1 bis 3, **dadurch gekennzeichnet, dass** zwecks Steigerung der katalytischen Leistung des Silbersalz-Katalysators auch ein Alkalimetall- oder quartäres Ammonium- oder Phosphoniumsalz einer Carbonsäure, vorzugsweise Natriumacetat, Malonsäuremonoethylester-Kaliumsalz, Maleinsäure-Dinatriumsalz bzw. Tetraethylammoniumacetat, verwendet wird.

5. Verfahren nach einem der Ansprüche 1 bis 4, **dadurch gekennzeichnet, dass** das Verfahren auch in Gegenwart von Triphenylphosphin durchgeführt wird.

6. Verfahren nach einem der Ansprüche 1 bis 3 und 5, **dadurch gekennzeichnet, dass** man pro Mol Edukt der Formel II etwa 0,05 bis etwa 0,3 Mol-% Silbersalz-Katalysator bzw. etwa 0,05 bis etwa 0,3 Mol-% quartäres Ammoniumsalz- oder Phosphoniumsalz-Katalysator bzw. bis zu etwa 0,2 Mol-% Triphenylphosphin verwendet.

7. Verfahren nach Anspruch 4, **dadurch gekennzeichnet, dass** man eine etwa äquimolare Menge des Alkalimetall- oder quartären Ammoniumoder Phosphoniumsalzes einer Carbonsäure bezüglich der Menge Silbersalz-Katalysator verwendet.

8. Verfahren nach einem der Ansprüche 1 bis 7, **dadurch gekennzeichnet, dass** es unter einem Kohlendioxid-Druck von etwa 1 bar bis etwa 30 bar (etwa 100 kPa bis etwa 3000 kPa) erfolgt.

9. Verfahren nach einem der Ansprüche 1 bis 8, **dadurch gekennzeichnet, dass** es im Temperaturbereich von etwa 25°C bis etwa 100°C erfolgt.

## Claims

1. A process for the manufacture of 4,4-disubstituted 5-methylene-1,3-dioxolan-2-ones of the general formula 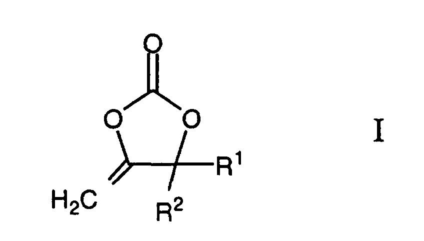 wherein R¹ and R² each independently signify a saturated or olefinically-unsaturated aliphatic group or an optionally substituted
aromatic group, or R¹ and R² together form tetra- or pentamethylene, by reacting a corresponding 3,3-disubstituted prop-1-yn-3-ol of the general formula 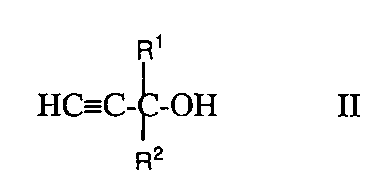 with carbon dioxide in the presence of a quaternary ammonium or phosphonium salt as the catalyst, which process comprises using a silver salt as an additional catalyst.

2. A process according to claim 1, wherein silver acetate, silver sulphate, silver orthophosphate, silver oxide or silver carbonate, preferably silver acetate or silver sulphate, is used as the silver salt catalyst.

3. A process according to claim 1 or 2, wherein tetramethylammonium chloride, tetraethylammonium chloride tetraethylammonium bromide or benzyltrimethylammonium chloride is used as the quaternary ammonium salt or tetrabutylphosphonium bromide, tetraphenylphosphonium chloride or tetraphenylphosphonium bromide is used as the quaternary phosphonium salt.

4. A process according to any one of claims 1 to 3, wherein in addition an alkali metal or quaternary ammonium or phosphonium salt of a carboxylic acid, preferably sodium acetate, potassium monoethyl malonate, disodium maleate or tetraethylammonium acetate, is used to increase the catalytic performance of the silver salt catalyst.

5. A process according any one of claims 1 to 4, wherein the reaction is also carried out in the presence of triphenylphosphine.

6. A process according to any one of claims 1 to 3 and 5, wherein about 0.05 to about 0.3 mol% silver salt catalyst and, respectively, about 0.05 to about 0.3 mol% quaternary ammonium salt or phosphonium salt catalyst and, respectively, up to about 0.2 mol% triphenylphosphine are used per mol of educt of formula II.

7. A process according to claim 4, wherein about an equimolar amount of the alkali metal or quaternary ammonium or phosphonium salt of a carboxylic acid relative to the amount of silver salt catalyst is used.

8. A process according to any one of claims 1 to 7, wherein the reaction is effected under a carbon dioxide pressure of about 1 bar to about ' 30 bar (about 100 kPa to about 3000 kPa).

9. A process according to any one of claims 1 to 8, wherein the reaction is effected in a temperature range of about 25°C to about 100°C.

## Revendications

1. Procédé de préparation de 5-méthylène-1,3-dioxolanne-2-ones 4,4-disubstituées de formule générale 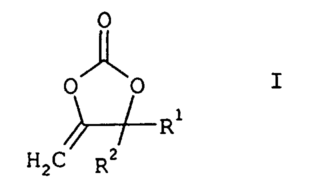 dans laquelle R¹ et R² représentent chacun indépendamment de l'autre un groupe aliphatique saturé ou à insaturation oléfinique, ou un groupe aromatique éventuellement substitué, ou encore R¹ et R² forment ensemble un radical tétra- ou pentaméthylène,
par réaction d'un prop-1-yne-3-ol 3,3-disubstitué correspondant de formule générale 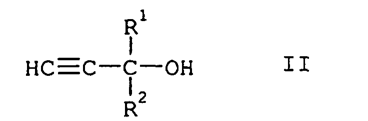 avec du dioxyde de carbone en présence d'un sel de l'ammonium ou du phosphonium quaternaire servant de catalyseur, **caractérisé en ce qu'**on utilise en tant que catalyseur supplémentaire un sel d'argent.

2. Procédé selon la revendication 1, **caractérisé en ce qu'**on utilise en tant que catalyseur au sel d'argent l'acétate d'argent, le sulfate d'argent, l'orthophosphate d'argent, l'oxyde d'argent ou le carbonate d'argent, de préférence l'acétate d'argent ou le sulfate d'argent.

3. Procédé selon la revendication 1 ou 2, **caractérisé en ce qu'**on utilise en tant que sel de l'ammonium quaternaire le chlorure de tétraméthylammonium, le chlorure de tétraéthylammonium, le bromure de tétraéthylammonium ou le chlorure de benzyltriméthylammonium, ou en tant que sel du phosphonium quaternaire le bromure de tétrabutylphosphonium, le chlorure de tétraphénylphosphonium ou le bromure de tétraphénylphosphonium.

4. Procédé selon l'une des revendications 1 à 3, **caractérisé en ce que**, pour augmenter l'activité catalytique du catalyseur à base d'un sel d'argent, on utilise aussi un sel d'un métal alcalin ou de l'ammonium quaternaire ou du phosphonium quaternaire d'un acide carboxylique, de préférence l'acétate de sodium, le sel de potassium de l'ester monoéthylique de l'acide malonique, le sel disodique de l'acide maléique ou l'acétate de tétraéthylammonium.

5. Procédé selon l'une des revendications 1 à 4, **caractérisé en ce que** le procédé est aussi mis en oeuvre en présence de triphénylphosphine.

6. Procédé selon l'une des revendications 1 à 3 et 5, **caractérisé en ce qu'**on utilise par mole de matière de départ de formule II d'environ 0,05 à environ 0,3 % en moles du catalyseur à base d'un sel d'argent, ou d'environ 0,05 à environ 0,3 % en moles du catalyseur à base du sel de l'ammonium quaternaire ou du sel du phosphonium quaternaire, ou encore jusqu'à environ 0,2 % en moles de triphénylphosphine.

7. Procédé selon la revendication 4, **caractérisé en ce qu'**on utilise une quantité approximativement équimolaire du sel d'un métal alcalin ou de l' ammonium ou du phosphonium quaternaires d'un acide carboxylique, par rapport à la quantité du catalyseur à base d'un sel d'argent.

8. Procédé selon l'une des revendications 1 à 7, **caractérisé en ce qu'**il est mis en oeuvre sous une pression de dioxyde de carbone d'environ 1 bar à environ 30 bar (d'environ 100 kPa à environ 3000 kPa).

9. Procédé selon l'une des revendications 1 à 8, **caractérisé en ce qu'**il est mis en oeuvre dans la plage de températures allant d'environ 25 à environ 100°C.
